# EUROPÄISCHE PATENTSCHRIFT

(11) **EP 3 984 431 B1**
(45) Veröffentlichungstag und Bekanntmachung des Hinweises auf die Patenterteilung: **19.06.2024**
(21) Anmeldenummer: 20201554.1
(22) Anmeldetag: 13.10.2020
(51) Int. Cl.: A47L 9/18

(54) **REINIGUNGSVORRICHTUNG SOWIE VERWENDUNG**
CLEANING DEVICE AND ITS USE
DISPOSITIF DE NETTOYAGE AINSI QU'UTILISATION

(43) Veröffentlichungstag der Anmeldung: 20.04.2022
(73) Patentinhaber: ReBerni Limited, Kwai Chung, New Territories (HK)
(72) Erfinder: Hug, Bernhard, 8408 Winterthur (CH)
(74) Vertreter: Patent- und Rechtsanwälte Behrmann Wagner PartG mbB

(56) Entgegenhaltungen:
- KR-A- 20120 061 784
- US-A- 979 211
- US-A- 5 199 963
- US-A1- 2019 151 788

## Beschreibung

Die vorliegende Erfindung betrifft eine Reinigungsvorrichtung zum Ansaugen eines mit Partikeln, Aerosolen, Bakterien und/oder Viren beladenen Saugluftvolumenstroms nach dem Oberbergriff des Anspruchs 1. Ferner betrifft die vorliegende Erfindung die Verwendung der erfindungsgemäßen Reinigungsvorrichtung als Umgebungsluftreinigungsvorrichtung und/oder als Nasssauger.

Aus dem Stand der Technik sind Reinigungsvorrichtungen mit einer Flüssigkeitsaufnahmeeinheit zum Ansaugen und Reinigen eines mit Partikeln beladenen Saugluftvolumenstroms allgemein bekannt.

Die bekannten Reinigungsvorrichtungen umfassen Unterdruckerzeugungsmittel, um über eine Luftaufnahmeöffnung den Saugluftvolumenstrom zusammen mit den Partikeln anzusaugen. Über ein Tauchrohr wird der mit den Partikeln beladene Saugluftvolumenstrom dann in eine Flüssigkeit geleitet, die von einer Flüssigkeitsaufnahmeeinheit aufgenommen wird. Beim Durchströmen der Flüssigkeit entlang einer sich ausbildenden Durchströmstrecke kommt es zu einem Reinigen des Saugluftvolumenstroms, da die mitgeführten Partikel größtenteils in der Flüssigkeit gebunden und somit dort zurückgehalten werden können.

Die KR 2012 0061 784 A lehrt bereits einen miniaturisierten wasserreinigenden Staubabscheider. Um eine Fehlfunktion zu verhindern, wird sichergestellt, dass kein Wasser außerhalb des Staubabscheiders austritt.

Aus der US 2019/151788 A1 ist eine Filteranordnung und ein Staubsauger mit derselben bekannt. Die Filteranordnung umfasst einen Tank und ein Lufteinlassrohr. Der Tank ist so gestaltet, dass er eine Flüssigkeit aufnehmen kann, und hat einen Lufteinlass und einen Luftauslass. Das Lufteinlassrohr ist mit dem Lufteinlass verbunden, hat ein Luftauslassende, das sich in dem Tank befindet und sich in Richtung der Flüssigkeit erstreckt oder in die Flüssigkeit eingetaucht ist, und hat eine Querschnittsfläche, die entlang einer Fluidströmungsrichtung innerhalb des Lufteinlassrohrs allmählich zunimmt

Ferner lehrt die US 979 211 A bereits eine Staubabscheidevorrichtung bzw. eine Staubsaugervorrichtung.

Weiterhin ist aus der US 5 199 963 A Staubsauger bekannt, der Luft behandelt, indem er die Luft durch ein Flüssigkeitsbad drückt, um suspendierte Partikel in der Luft zu entfernen. Der Staubsauger umfasst ein Schalenelement, das zur Aufnahme des Flüssigkeitsbades geeignet ist, einen Reinigungslufteinlass, ein oberes Gehäuse, das von dem Schalenelement getragen wird und an diesem befestigt ist, und einen auf dem oberen Gehäuse montierten Motor mit einem Kühllufteinlass und einem Kühlluftauslass, der von dem der Reinigungsluft getrennt ist. Der Reinigungslufteinlass wird von einem Hauptleitblech umschlossen, das den Überspritzungseffekt auffängt, der durch die von der einströmenden Reinigungsluft im Flüssigkeitsbad erzeugten Flüssigkeitsturbulenzen hervorgerufen wird.

Von Nachteil ist hierbei eine hohe Strömungsgeschwindigkeit des Saugluftvolumenstroms beim Durchströmen der Flüssigkeit, da sich hierbei große Blasen ausbilden und die Flüssigkeit schnell durchströmt wird, was für das Zurückhalten der Partikel in der Flüssigkeit und somit für eine gute Reinigungswirkung gattungsbildender Reinigungsvorrichtungen nachteilig ist.

Aus diesem Grund ist es Aufgabe der vorliegenden Erfindung, die aus dem Stand der Technik bekannten Nachteile einer Reinigungsvorrichtung nach dem Oberbegriff des Anspruchs 1 zu überwinden. Insbesondere ist es Aufgabe der vorliegenden Erfindung eine Reinigungsvorrichtung anzugeben, die auch bei einer weiten Variation des Saugluftvolumenstroms eine gute Reinigungswirkung erzielt.

Die Aufgabe der vorliegenden Erfindung wird durch eine Reinigungsvorrichtung nach Anspruch 1 gelöst.

Die Erfindung sieht vor, eine Reinigungsvorrichtung zum Ansaugen eines mit Partikeln, Aerosolen, Bakterien und/oder Viren beladenen Saugluftvolumenstroms aus einer Umgebung anzugeben, aufweisend eine einen ersten Leitungsquerschnitt umfassende Luftaufnahmeöffnung zum Ansaugen des mit den Partikeln beladenen Saugluftvolumenstroms aus der Umgebung, eine ein Tauchrohr umfassende Flüssigkeitsaufnahmeeinheit zum Aufnehmen von einer Flüssigkeit, insbesondere von Wasser, wobei das Tauchrohr an einem ersten Ende mit der Luftaufnahmeöffnung saugluftleitend verbunden ist und so ausgebildet ist, dass es an einem zweiten Ende in die aufnehmbare Flüssigkeit, insbesondere in das Wasser, bereichsweise eintaucht, derartig, dass in einem aktiven Betriebszustand der Reinigungsvorrichtung die aufnehmbare Flüssigkeit von dem mit den Partikeln beladenen Saugluftvolumenstrom zum Zurückhalten der Partikel durchströmt wird, sowie Unterdruckerzeugungsmittel zum Erzeugen des Saugluftvolumenstroms in dem aktiven Betriebszustand. Erfindungsgemäß ist vorgesehen, dass ein zweiter Leitungsquerschnitt des Tauchrohrs zum Führen des Saugluftvolumenstroms wenigstens an dem zweiten Ende größer ist als der erste Leitungsquerschnitt der Luftaufnahmeöffnung.

Anders ausgedrückt, die erfindungsgemäße Reinigungsvorrichtung umfasst eine Luftaufnahmeöffnung mit einem ersten Leitungsquerschnitt, die zum Ansaugen eines Luft-Partikel-Gemisches aus einer Umgebung ausgebildet ist, wobei unter dem Begriff von Partikeln im Rahmen der vorliegenden Erfindung auch Staub, bevorzugt Ultra-Feinstaub, Feinstaub und/oder Grobstaub, Aerosolpartikel, Bakterien und/oder Viren mitumfasst sein sollen. Der angesaugte, mit den Partikeln beladene Saugluftvolumenstrom wird durch Unterdruckerzeugungsmittel gebildet, die von der Reinigungsvorrichtung umfasst sind.

Ferner umfasst die erfindungsgemäße Reinigungsvorrichtung eine Flüssigkeitsaufnahmeeinheit, die ein erfindungsgemäßes Tauchrohr aufweist und zum Aufnehmen einer Flüssigkeit, insbesondere einer Reinigungsflüssigkeit, ausgebildet ist.

Das Tauchrohr ist einends, insbesondere unmittelbar oder über eine Zuleitung mittelbar, mit der Ansaugöffnung saugluftleitend verbunden und mündet anderenends in der aufnehmbaren Flüssigkeit, damit sichergestellt wird, dass der mit den Partikeln beladene Saugluftvolumenstrom die Flüssigkeit entlang einer Durchströmstrecke durchströmt, um die Partikel in einer durch die Flüssigkeit ausgebildeten ersten Filterstufe vom Saugluftvolumenstrom zu trennen und in der Flüssigkeit zu binden.

Erfindungsgemäß ist es nun vorgesehen, dass das Tauchrohr zumindest an dem in die Flüssigkeit eintauchenden Ende einen zweiten Leitungsquerschnitt aufweist, der in Bezug auf den ersten Leitungsquerschnitt der Luftaufnahmeöffnung größer ausgebildet ist.

Vorteilhaft führt die Querschnittserweiterung von der eingangsseitig angeordneten Luftaufnahmeöffnung zu dem ausgangsseitig angeordneten Tauchrohr der ersten Filterstufe zu einem Verlangsamen der Strömungsgeschwindigkeit des in einem aktiven Betriebszustand der Reinigungsvorrichtung erzeugten Saugluftvolumenstroms, weshalb sich beim Durchströmen der Flüssigkeit kleinere Luftblasen ausbilden und die Verweildauer des Saugluftvolumenstroms beim Durchströmen der aufnehmbaren Flüssigkeit aufgrund der reduzierten Strömungsgeschwindigkeit erhöht werden kann. Somit wird vorteilhaft die Reinigungswirkung verbessert, da verhältnismäßig mehr Partikel in der Flüssigkeit zurückgehalten werden können.

Vorteilhaft wirkt sich die erfindungsgemäße interne Erweiterung des Leitungsquerschnitts nicht auf den zum Ansaugen der Partikel und/oder des Partikel-Luft-Gemisches benötigten Saugluftvolumenstrom an der Luftaufnahmeöffnung aus, weshalb dort in Bezug auf die Strömungsgeschwindigkeit zum Ansaugen von Partikeln eine ausreichend große Ansaugleistung erzielt werden kann.

Zum Ausbilden der ersten Reinigungsstufe wird im Rahmen der vorliegenden Erfindung als Reinigungsflüssigkeit für die Flüssigkeitsaufnahmeeinheit bevorzugt Wasser oder Leitungswasser eingesetzt, um einen schnellen Austausch und eine einfache Entsorgung bei starker Verschmutzung der Flüssigkeit zu ermöglichen.

Ferner wird darauf hingewiesen, dass als ein Ruhezustand der Reinigungsvorrichtung ein passiver Betriebszustand der Reinigungsvorrichtung verstanden wird, bei dem die Flüssigkeitsaufnahmeeinheit Flüssigkeit, insbesondere Wasser, so aufnimmt, dass das Tauchrohr endseitig wenigstens bereichsweise in die aufnehmbare Flüssigkeit eintaucht.

In dem Ruhezustand wird durch die Unterdruckerzeugungsmittel kein Saugluftvolumenstrom erzeugt, weshalb es nicht zum Ausbilden von Turbulenzen, Verwirbelungen und/oder Luftblasen, insbesondere entlang der Durchströmstrecke, in der aufnehmbaren Flüssigkeit kommt.

In einem aktiven Betriebszustand der Reinigungsvorrichtung kommt es dagegen zu einem Ansaugen eines Partikel-Luft-Gemisches durch den mittels der Unterdruckerzeugungsmittel erzeugbaren Saugluftvolumenstrom. Ferner strömt in dem aktiven Betriebszustand der mit den Partikeln beladene Saugluftvolumenstrom durch das Tauchrohr in die aufnehmbare Flüssigkeit ein, weshalb es dort zum Ausbilden von Turbulenzen, Wirbeln und/oder Strudeln kommt, wobei die Flüssigkeit entlang einer Durchströmstrecke durchströmt wird und wobei es zum Zurückhalten und Fixieren von vom erzeugten Saugluftvolumenstrom mitgeführten Partikeln kommt.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

In einer bevorzugten Ausführungsform der vorliegenden erfindungsgemäßen Reinigungsvorrichtung besteht ein Verhältnis zwischen dem zweiten Leitungsquerschnitt des Tauchrohrs und dem ersten Leitungsquerschnitt der Luftaufnahmeöffnung von wenigstens 2, bevorzugt wenigstens 3, weiter bevorzugt wenigstens 3,5.

Mit anderen Worten soll das Verhältnis des Durchmessers oder des Querschnitts des Tauchrohrs am zweiten Ende zum Durchmesser oder Querschnitt der Luftaufnahmeöffnung größer gleich 2, bevorzugt größer gleich 3, weiter bevorzugt größer gleich 3,5 sein, um somit eine Beeinflussung der Strömungsgeschwindigkeit des Saugluftvolumenstroms zu realisieren. Vorteilhaft führt die sprungartige, kontinuierliche und/oder schrittweise Erhöhung des Leitungsquerschnitts von der Luftaufnahmeöffnung zum zweiten Ende des Tauchrohrs zu einer Verringerung der mittleren Strömungsgeschwindigkeit des Saugluftvolumenstroms, wobei sich bei einem derart gewählten Verhältnis eine besonders effiziente Reinigung (hohe Reinigungswirkung) des mit den Partikeln beladenen Saugluftvolumenstroms beim Durchströmen der Flüssigkeit entlang der Durchströmstrecke erzielen lässt.

Zudem ist bei einem derartigen Verhältnis sichergestellt, das zum einen eine hohe Reinigungswirkung beim Durchströmen der Flüssigkeit erzielt werden kann und zum anderen noch eine ausreichende Ansaugleistung zum Ansaugen des Partikel-Luft-Gemisches durch die Luftaufnahmeöffnung ermöglicht wird.

Gemäß einem weiteren Beispiel umfasst das Tauchrohr wenigstens einen bogen- und/oder winkelförmigen Umlenkabschnitt und einen, insbesondere entlang einer Vertikalachse ausgerichteten, Absenkabschnitt, wobei der Umlenkabschnitt so ausgebildet ist, dass in dem aktiven Betriebszustand der mit den Partikeln beladene Saugluftvolumenstrom in Bezug auf eine Einströmrichtung umgelenkt und verwirbelt wird und sich somit eine erste Beeinflussung der Strömungsgeschwindigkeit realisieren lässt.

Ferner ist es in diesem Zusammenhang bevorzugt vorgesehen, wenn das Tauchrohr zweiteilig oder mehrteilig ausgebildet ist. Eine zwei- oder mehrteilige Ausgestaltung des Tauchrohrs ist besonders vorteilhaft, wenn sich somit ein Klappe und/oder ein Deckel aus dem Tauchrohr lösen lässt, um die im Grobfilter gesammelten Partikel in einem passiven Betriebszustand der Reinigungsvorrichtung zu entnehmen und somit die Reinigung des Grobfilters zu erleichtern.

Ferner ist der Absenkabschnitt bevorzugt so ausgebildet, dass der in dem aktiven Betriebszustand der Reinigungsvorrichtung im Umlenkabschnitt umgelenkte, verwirbelte sowie verlangsamte Saugluftvolumenstrom durch eine sprungartige, schrittweise oder kontinuierliche Erweiterung des Leitungsquerschnitts in Bezug auf eine mittlere Einströmgeschwindigkeit zusätzlich verlangsamt wird.

Zudem ist der Absenkabschnitt in Bezug auf seine Länge (Längserstreckung) so dimensioniert, dass sich die ausgebildeten Verwirbelungen größtenteils vor dem Einströmen in die Flüssigkeit zurückbilden und abklingen. Vorteilhaft wird hierdurch eine Verlangsamung des Saugluftvolumenstroms sowie eine homogene Verteilung der Partikel realisiert, die sich im gesamten Leitungsquerschnitt des Tauchrohrs auswirkt

Weiterbildend ist es vorgesehen, dass der Umlenkabschnitt dahingehend ausgebildet ist, dass in dem aktiven Betriebszustand der mit den Partikeln beladene Saugluftvolumenstrom in Bezug auf die durch die Luftaufnahmeöffnung definierte Einströmrichtung um einen Umlenkwinkel β von mehr als 60°, bevorzugt mehr als 70°, besonders bevorzugt mehr als 80°, ganz besonders bevorzugt im Wesentlichen 90° umgelenkt wird.

Vorteilhaft kann somit durch die Umlenkung des Saugluftvolumenstroms eine erste Reduzierung der Strömungsgeschwindigkeit des Saugluftvolumenstroms realisiert werden, da der Saugluftvolumenstrom im Umlenkabschnitt wenigstens teilweise gegen die Innenwandung des Tauchrohrs prallt.

Zudem wird darauf hingewiesen, dass unter einem Umlenkwinkel β von etwa 90° insbesondere ein Bereich verstanden wird, der bevorzugt zwischen 88° und 92° liegt.

Ferner ist es gemäß einem weiteren Beispiel vorgesehen, dass das Tauchrohr so innerhalb der Flüssigkeitsaufnahmeeinheit angeordnet ist, dass sich der Absenkabschnitt in Vertikalrichtung erstreckt. Vorteilhaft führt ein sich entlang der Vertikalachse erstreckender Absenkabschnitt dazu, dass der mit den Partikeln beladene Saugluftvolumenstrom quer zu einer von der Flüssigkeit im passiven Betriebszustand ausbildbaren Flüssigkeitsoberfläche strömt und somit auch quer, also rechtwinklig zu dieser Flüssigkeitsoberfläche in die Flüssigkeit einströmt.

In diesem Zusammenhang wird darauf hingewiesen, dass im aktiven Betriebszustand die Flüssigkeit keine ebene Flüssigkeitsoberfläche ausbildet, da es durch den einströmenden Saugluftvolumenstrom zum Entstehen von Verwirbelungen, Strudeln und/oder sich überlagernden Wellen in der aufnehmbaren Flüssigkeit der Flüssigkeitsaufnahmeeinheit kommt. Dennoch kann die Durchströmstrecke des Saugluftvolumenstroms beim Durchströmen der Flüssigkeit durch das vertikale Zuleiten des Saugluftvolumenstroms in die aufnehmbare Flüssigkeit bezüglich der Reinigungswirkung positiv beeinflusst werden.

Mit anderen Worten, das Tauchrohr ist bevorzugt so in der Flüssigkeitsaufnahmeeinheit angeordnet, dass die Längserstreckung des Tauchrohrs zumindest im Bereich des zweiten Endes quer oder rechtwinklig zu einer im Ruhezustand ausgebildeten Flüssigkeitsoberfläche ausgerichtet ist, sich also entlang einer Vertikalachse erstreckt. Vorteilhaft führt dies dazu, dass die aufgrund ihres Eigengewichts auf die Partikel wirkende Gewichtskraft längs zu der Strömungsrichtung des Saugluftvolumenstroms ausgerichtet ist. Ferner muss der Saugluftvolumenstrom somit in Bezug auf seine Einströmrichtung eine Umlenkung von 180° durchlaufen, um wieder aus der Flüssigkeit herauszuströmen. Vorteilhaft wirkt sich dies positiv auf die Reinigungswirkung aus.

Erfindungsgemäß umfasst die Reinigungsvorrichtung eine zweite Filterstufe, die durch einen gitterartigen Grobfilter zum Zurückhalten von großen Partikeln ausgebildet ist. Der Grobfilter ist bevorzugt in einem Bereich des Tauchrohrs ausgebildet und/oder angeordnet, der vollständig in die Flüssigkeit eingetaucht ist. Bevorzugt ist der Grobfilter durch eine Vielzahl an stabförmigen Zinken ausgebildet, die jeweils voneinander beabstandet angeordnet sind und sich längs zu der Erstreckungsachse des Tauchrohrs, insbesondere längs zur der Längserstreckung des Absenkabschnitts, also insbesondere in Vertikalrichtung, erstecken. Durch die Zinken werden in der Mantelfläche des Tauchrohrs spaltartige Öffnungen für den Saugluftvolumenstrom ausgebildet, die dann wiederum große Partikel zurückhalten und innerhalb des Tauchrohrs fixieren und sammeln, da sie nicht durch die spaltartigen Öffnungen passen. Mit großen Partikeln werden in diesem Zusammenhang Partikelgrößen verstanden, die ein Längs-, Quer-, und/oder Breitenerstreckung von größer als 2mm umfassen.

Neben dem Zurückhalten der großen Partikel innerhalb des Tauchrohrs wird durch den Grobfilter zusätzlich auch die Reinigungswirkung von kleineren Partikeln beim Durchströmen der Flüssigkeit verbessert, da durch große Partikel, die frei in der Flüssigkeit herumschwimmen, die Reinigungswirkung für kleine Partikel, also insbesondere Partikel mit einer Längs-, Quer- und/oder Breitenerstreckung von kleiner als 2mm, negativ beeinflusst wird.

Ferner ist es in diesem Zusammenhang bevorzugt vorgesehen, dass das Tauchrohr an dem zweiten Ende eine Stirnseite aufweist, die mit einem Bodenelement der Flüssigkeitsaufnahmeeinheit verbunden ist, derartig, dass in dem aktiven Betriebszustand der Saugluftvolumenstrom ausschließlich durch die vom Grobfilter ausgebildeten Öffnungen strömen kann.

Somit erstreckt sich das Tauchrohr insbesondere bis zu dem Bodenelement der Flüssigkeitsaufnahmeeinheit, wobei die Stirnseite des Tauchrohrs bevorzugt mit dem Bodenelement verbunden ist. Damit kann der mit den Partikeln beladene Saugluftvolumenstrom nur durch die seitlichen, in der Mantelfläche des Tauchrohrs ausgebildeten Öffnungen des Grobfilters aus dem Tauchrohr herausströmen. Alternativ kann das Tauchrohr endseitig auch durch ein, insbesondere scheibenförmiges, Abschlusselement verschlossen sein, um sicherzustellen, dass der Saugluftvolumenstrom vollständig durch die Öffnungen des Grobfilters aus dem Tauchrohr strömt.

Weiterbildend umfasst die Reinigungsvorrichtung einen Tropfenabscheider, der zum Zurückhalten von vom Saugluftvolumenstrom aufgenommenen Flüssigkeitstropfen und weiteren Partikeln saugluftleitend zwischen der Flüssigkeitsaufnahmeeinheit und den Unterdruckerzeugungsmitteln angeordnet ist

Vorteilhaft realisiert der Tropfenabscheider eine dritte Filterstufe der Reinigungsvorrichtung, da nach dem Durchströmen der Flüssigkeit zum Zurückhalten von Partikeln (erste Filterstufe) und dem Durchströmen des Grobfilters zum Zurückhalten von großen Partikeln (zweite Filterstufe) auch durch den Tropfenabscheider Partikel aus dem Saugluftvolumenstrom herausgefiltert werden (dritte Filterstufe). Neben dem Herausfiltern von Partikeln werden im Tropfenabscheider aber auch Flüssigkeitstropfen vom Saugluftvolumenstrom getrennt, die zuvor vom Saugluftvolumenstrom beim Durchströmen der Flüssigkeit aufgenommen wurden.

Vorteilhaft ist der Tropfenabscheider in Bezug auf die Flüssigkeitsaufnahmeeinheit in einem Bereich angeordnet, der es ermöglicht, dass die aus dem Saugluftvolumenstrom absorbierten Flüssigkeitstropfen wieder in die Flüssigkeitsaufnahmeeinheit zurückgeleitet werden. Vorteilhaft verhindert dies einen schnellen Verlust von Flüssigkeit im Betrieb der Reinigungsvorrichtung, weshalb länger Betriebszeiten der Reinigungsvorrichtung realisierbar sind. Ferner kann somit die Reinigungswirkung zusätzlich verbessert werden, da neben dem Absorbieren von Flüssigkeitstropfen auch Partikel aus dem Saugluftvolumenstrom herausgelöst werden können, die nicht beim Durchströmen Flüssigkeitsaufnahmeeinheit in der Flüssigkeit gebunden werden konnten.

Weiterbildend umfasst der Tropfenabscheider einen Anströmbereich, der durch eine Vielzahl von profilierten, voneinander beabstandeten, insbesondere blechartigen, Lamellen ausgebildet ist, wobei zwischen zwei benachbarten Lamellen jeweils ein Durchströmkanal mit wenigstens zwei Umlenkbereichen für den Saugluftvolumenstrom ausgebildet ist und/oder wobei der Anströmbereich in einem Fixierwinkel zwischen 20° bis 70°, bevorzugt 30° bis 60°, besonders bevorzugt 40° bis 50°, ganz besonders bevorzugt im Wesentlichen 45° zu einer Horizontalebene, insbesondere zu einer Flüssigkeitsoberfläche der von der Flüssigkeitsaufnahmeeinheit aufnehmbaren Flüssigkeit ausgerichtet ist.

Vorteilhaft und weiterbildend ist der Anströmbereich durch eine vom Saugluftvolumenstrom angeströmte Fläche ausgebildet, von der eine Vielzahl von schlitzartigen Passagen für den Saugluftvolumenstrom ausgeht. Der Saugluftvolumenstrom strömt im aktiven Betriebszustand auf den winklig zu einer Horizontalebene ausgerichteten Anströmbereich, weshalb es zur Aufteilung des Saugluftvolumenstroms in eine Vielzahl an Teilströmen kommt, die jeweils entlang einer zwischen den Lamellen gebildeten Passage strömen (Durchströmkanal). Die Passagen umfassen Umlenkbereiche, die durch eine bogenförmige Krümmung der Lamellen ausgebildet werden und somit Unterdruckbereiche ausbilden. Vorteilhaft führt die Umlenkung der Teilströme dazu, dass aufgrund der auf die Flüssigkeitstropfen sowie weitere Partikel wirkenden Trägheit diese von den Teilströmen absorbiert werden.

Vorteilhaft ist der Tropfenabscheider ferner in einem Winkel zu einer Horizontalebene ausgerichtet, der dazu führt, dass die absorbierten Flüssigkeitstropfen aufgrund der Gewichtskraft zurück in die Flüssigkeitsaufnahmeeinheit fließen. Ein schnelles Ableiten der Flüssigkeitstropfen führt ferner dazu, dass es nicht zu einer erneuten Aufnahme der Flüssigkeitstropfen kommt. Vorteilhaft führt dies zu einem Reduzieren der vom Saugluftvolumenstrom aufgenommenen Feuchtigkeit und/oder der Anzahl der enthaltenen Flüssigkeitstropfen, die ausgangsseitig der Reinigungsvorrichtung durch eine Luftabgabeöffnung wieder an die Umgebung abgeführt wird/werden.

Mit anderen Worten, der Topfenabscheider sowie die ausgebildeten Durchströmkanäle sind so ausgerichtet und/oder ausgebildet, dass die Flüssigkeitstropfen nach dem Trennen von dem Saugluftvolumenstrom wegen der auf sie wirkenden Gewichtskraft von den Durchströmkanälen wegführbar sind. Bevorzugt ist es in diesem Zusammenhang vorgesehen, dass die Flüssigkeitstropfen anschließend wieder in die Flüssigkeitsaufnahmeeinheit zurückgeleitet werden, um einen Flüssigkeitsverlust im aktiven Betriebszustand der Reinigungsvorrichtung zu vermeiden.

Weiterbildend ist es vorgesehen, dass der Tropfenabscheider statisch und/oder unbeweglich ausgebildet ist und insbesondere keine rotierenden Elemente umfasst. Vorteilhaft führt dies zu der Realisierung einer in Bezug auf den Energieverbrauch effizienten Reinigungsvorrichtung. Ferner lässt sich somit insbesondere eine Reinigungsvorrichtung angeben, die sich im Betrieb durch eine geringe Geräuschkulisse auszeichnet und somit im Allgemeinen als leise und somit nicht-störend von anwesenden Personen aufgefasst wird.

Weiterbildend ist zudem vorgesehen, dass die Reinigungsvorrichtung eine Filtereinheit zum Herausfiltern von vom Saugluftvolumenstrom mitgeführten Feinpartikeln (Restpartikeln) umfasst, die insbesondere im Saugluftpfad in einer Kanalpassage saugluftleitend zwischen dem Tropfenabscheider und den Unterdruckerzeugungsmitteln angeordnet ist. Vorteilhaft ermöglicht dies eine weitere Verbesserung der erfindungsgemäßen Reinigungsvorrichtung, um die Filterwirkung der Reinigungsvorrichtung in Bezug auf Feinstaub zu verbessern. Ferner ermöglicht dies vorteilhaft, dass die Reinigungsvorrichtung um eine vierte Reinigungsstufe erweitert wird, um die Reinigungswirkung der vorliegenden Reinigungsvorrichtung zusätzlich zu verbessern.

Weiterbildend umfasst die Filtereinheit zudem eine Aufnahmeeinheit, die zur Aufnahme eines austauschbaren Gewebefilters ausgebildet ist. Beim Durchströmen des Gewebefilters kann der Saugluftvolumenstrom zusätzlich von Feinstaub und/oder Ultra-Feinstaub befreit werden, da sich dieser beim Durchströmen des Gewebefilters in Abhängigkeit einer wählbaren Porosität im Gewebefilter festsetzt. Durch den Einsatz von Gewebefiltern mit unterschiedlicher Porosität ist somit auch eine anwendungsspezifische Optimierung möglich.

Gemäß einem weiteren Beispiel sind zudem UV- und/oder UVC-Lichterzeugungsmittel vorgesehen, die von der Reinigungsvorrichtung umfasst sind. Vorteilhaft sind die Lichterzeugungsmittel dahingehend in der Reinigungsvorrichtung angeordnet, dass der im aktiven Betriebszustand von dem erzeugten Saugluftvolumenstrom innerhalb der Reinigungsvorrichtung durchlaufende Saugluftpfad wenigstens bereichsweise, insbesondere entlang einer innenliegenden Passage, mit Lichtstrahlen eines bestimmten Wellenlängenbereichs, bevorzugt zwischen 100nm bis 450nm, weiter bevorzugt zwischen 100nm bis 280nm, ausgeleuchtet wird, derartig, dass der im aktiven Betriebszustand erzeugbare Saugluftvolumenstrom zum Töten von mitgeführten Viren und Keimen vollständig beleuchtet wird. Bevorzugt ist es hierbei vorgesehen, dass die Passage, innerhalb der die Beleuchtung erfolgt, als geschlossene Einheit ausgebildet ist, um insbesondere einen Austritt der (schädlichen) UVC-Strahlen an die Umgebung am Betriebsort der Reinigungsvorrichtung zu vermeiden.

Zudem ist es im Rahmen der vorliegenden Erfindung weiterbildend vorgesehen, dass die erfindungsgemäße Reinigungsvorrichtung als Luftreinigungsvorrichtung zur Reinigung von Umgebungsluft und/oder als Nassstaubsauger eingesetzt wird.

Weitere Vorteile Merkmale und Einzelheiten der Erfindung ergeben sich aus der nachfolgenden Beschreibung bevorzugter Ausführungsbeispiele sowie anhand der Zeichnungen. Diese zeigen in:
- Fig. 1:: eine perspektivische Ansicht einer bevorzugten Ausführungsform der erfindungsgemäßen Reinigungsvorrichtung, in
- Fig. 2:: eine Explosionsdarstellung der aus der Fig. 1 bekannten Reinigungsvorrichtung, in
- Fig. 3:: eine erste Schnittansicht der bekannten Reinigungsvorrichtung entlang einer Vertikalebene, in
- Fig. 4:: eine zweite Schnittansicht der bekannten Reinigungsvorrichtung entlang einer Horizontalebene, in
- Fig. 5:: eine dritte Schnittansicht des Flüssigkeitsabsorbers in der in der Fig. 3 dargestellten Perspektive B und in
- Fig. 6:: ein Teilausschnitt der Fig. 5 im vergrößerten Maßstab.

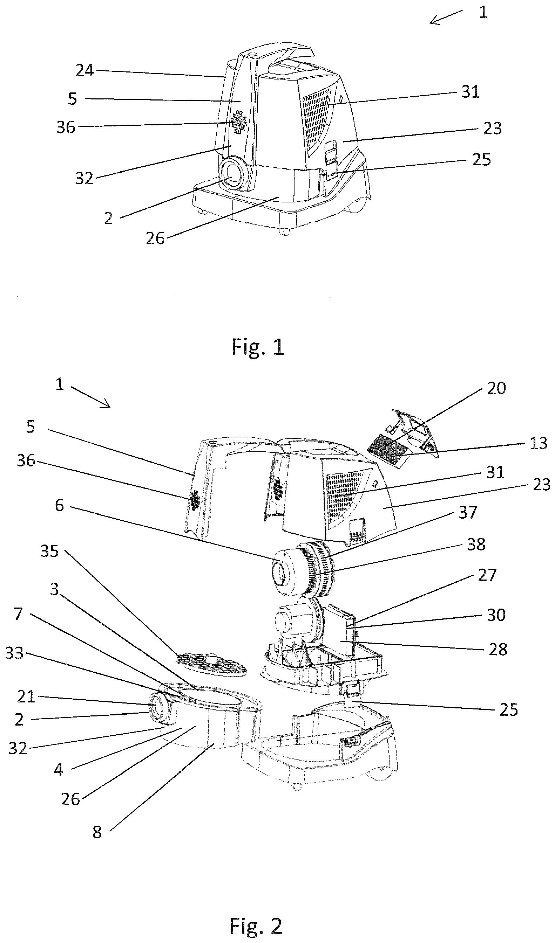

Die Fig. 1 zeigt eine erfindungsgemäße Reinigungsvorrichtung 1 zum Ansaugen eines mit Partikeln beladenen Saugluftvolumenstroms aus einer Umgebung in einer perspektivischen Ansicht nach einer ersten bevorzugten Ausführungsform.

Die dargestellte Reinigungsvorrichtung 1 umfasst eine Luftaufnahmeöffnung 2, ausgebildet zum Ansaugen des Saugluftvolumenstroms aus einer Umgebung in einem aktiven Betriebszustand der Reinigungsvorrichtung 1, wobei die Luftaufnahmeöffnung 2 bevorzugt mit einem nicht grafisch dargestellten Saugschlauch saugluftleitend verbindbar ist, um insbesondere einen Nassstaubsauger auszubilden.

Alternativ kann die Reinigungsvorrichtung 1 aber ohne Saugschlauch verwendet werden, um durch die Luftaufnahmeöffnung 2 unmittelbar Umgebungsluft aufzusaugen und somit eine Umgebungsluftreinigungsvorrichtung zum Herausfiltern von angesaugten Partikeln auszubilden.

Die Reinigungsvorrichtung 1 umfasst ein oberes Gehäuseelement 5 und ist auf vier Rollen angeordnet. An zwei gegenüberliegenden Seitenflächen 23, 24 des oberen Gehäuseelements 5 sind Verschlussmittel 25 angeordnet, die das obere Gehäuseelement 5 mit einem unteren Gehäuseelement 26 der Reinigungsvorrichtung 1 verbinden.

An der Vorderseite 32 der Reinigungsvorrichtung 1 ist im unteren Gehäuseelement 26 die Luftaufnahmeöffnung 2 angeordnet, durch die im aktiven Betriebszustand der Reinigungsvorrichtung 1 der Saugluftvolumenstrom, also ein aus der Umgebung angesaugtes Partikel-Luft-Gemisch, angesaugt wird. Das obere Gehäuseelement 5 umfasst eine Vielzahl an Lüftungsöffnung 36 (Perforationen) für einen Kühlkreislauf der Reinigungsvorrichtung 1.

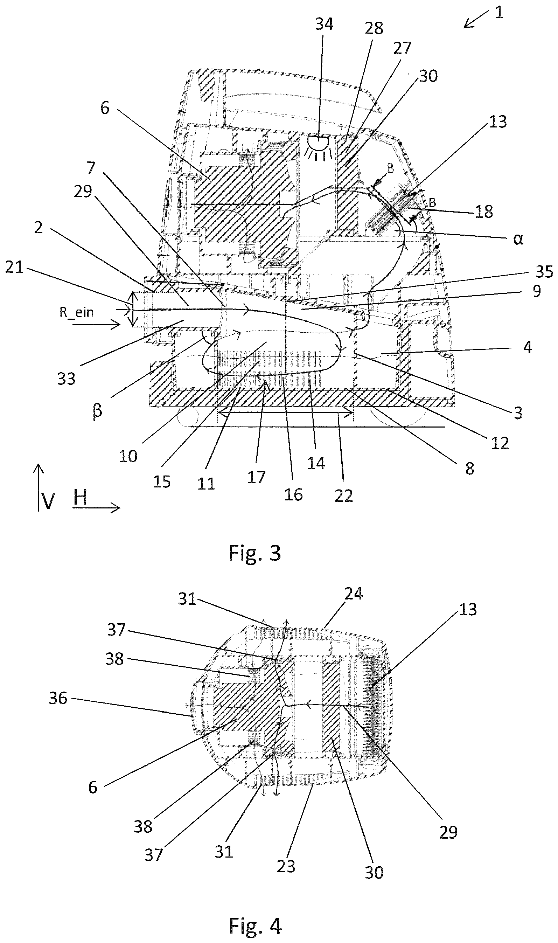

Ferner sind an den Seitenflächen 23, 24 Luftabgabeöffnungen 31 angeordnet, durch die der Saugluftvolumenstrom nach einer mehrstufigen Reinigung wieder an die Umgebung abgeführt wird. Der genaue Verlauf des mit den Partikeln beladenen Saugluftvolumenstroms sowie die unterschiedlichen Reinigungsstufen der erfindungsgemäßen Reinigungsvorrichtung 1 wird durch in Bezug auf die Fig. 3 und die Fig. 4 beschrieben.

In der Fig. 2 ist eine Explosionsdarstellung der aus der Fig. 1 bekannten Reinigungsvorrichtung 1 dargestellt. Aus der Darstellung geht hervor, dass das untere Gehäuseelement 26 der Reinigungsvorrichtung 1 durch eine Flüssigkeitsaufnahmeeinheit 4 ausgebildet wird, die eine Wanne zum Aufnehmen von Flüssigkeit sowie ein Tauchrohr 3 aufweist. Die an der Fronseite 32 ausgebildete Luftaufnahmeöffnung 2 zum Ansaugen des mit den Partikeln beladenen Saugluftvolumenstroms aus der Umgebung im aktiven Betriebszustand der Reinigungsvorrichtung 1 umfasst einen ersten Leitungsquerschnitt 21 und ist über eine Zuleitung 33 saugluftleitend mit einem ersten Ende 7 des Tauchrohrs 3 der Flüssigkeitsaufnahmeeinheit 4 verbunden. Das Tauchrohr 3 ragt an einem zweiten Ende 8 in die aufnehmbare Flüssigkeit ein, damit in dem aktiven Betriebszustand der Reinigungsvorrichtung 1 der mit den Partikeln beladenen Saugluftvolumenstrom die aufnehmbare Flüssigkeit entlang einer Durchströmstrecke zum Zurückhalten und Binden der Partikel in der Flüssigkeit durchströmt wird. Das Tauchrohr 3 umfasst ein Deckelelement 35 zum Ausbilden einer Reinigungsöffnung, um im Tauchrohr 3 zurückgehaltene Partikel manuell (durch Bedienpersonal) wieder zu entfernen.

Das Deckelelement 35 wird mittels nicht dargestellten Federmitteln durch das oberer Gehäuseelement 5 luftdicht auf das Tauchrohr 3 gepresst, wobei eine druckbeaufschlagte Arretierung des Deckelelements 35 im aktiven Betriebszustand der Reinigungsvorrichtung 1 durch die beidseitig angeordneten Verschlussmittel 25 realisiert wird. Vorteilhaft kann durch die luftdichte Anordnung des Deckelelements 35 auf dem Tauchrohr 3 das Ausbilden von Bypässen des Saugluftvolumenstroms verhindert werden, weshalb der gesamte Saugluftvolumenstrom die Flüssigkeit zum Zurückhalten von Partikeln im aktiven Betriebszustand durchströmt.

Ferner umfasst die dargestellte Reinigungsvorrichtung 1 Unterdruckerzeugungsmittel 6, die vorliegend durch einen Elektromotor ausgebildet sind, der in dem aktiven Betriebszustand der Reinigungsvorrichtung 1 durch Rotation den Saugluftvolumenstrom zum Ansaugen eines Luft-Partikel-Gemisches erzeugt. Der Elektromotor wird durch einen Kühlkreislauf mittels Umgebungsluft gekühlt, wobei der Kühlkreislauf die Luft durch die Öffnungen 36 im oberen Gehäuseelement 5 ansaugt.

Ferner umfassen die Unterdruckerzeugungsmittel 6 eine umfangsseitig ausgebildete Kühlkreislaufableitöffnung 38 zum Ableiten der angesaugten Luft des Kühlkreislaufs sowie eine umfangsseitig ausgebildete Saugluftvolumenstromableitöffnung 37 zum Ableiten des Saugluftvolumenstroms.

Vorteilhaft umfasst das obere Gehäuseelement 5 lediglich zwei Luftabgabeöffnungen 31, die jeweils durch eine Perforierung in den Seitenflächen 23, 24 der Reinigungsvorrichtung 1 angeordnet sind, um sowohl die Luft aus dem Kühlkreislauf als auch den gereinigten Saugluftvolumenstrom wieder an die Umgebung abzuleiten.

Ferner umfasst die Reinigungsvorrichtung 1 einen Tropfenabscheider 13, der von dem Saugluftvolumenstrom durchströmt wird, wobei sich aufgrund von vorgesehenen Durchströmkanälen 20 Flüssigkeitstropfen und weitere Partikel aus dem Saugluftvolumenstrom abschneiden lassen.

Zudem geht aus der Explosionsdarstellung hervor, dass eine weitere Filterstufe der Reinigungsvorrichtung 1 durch eine Filtereinheit 27 mit einer Aufnahmeeinheit 28 für einen Gewebefilter 30 ausgebildet wird. Der Gewebefilter 30 ist durch die Aufnahmeeinheit 28 so in einem Saugluftpfad des Saugluftvolumenstroms angeordnet, dass der Gewebefilter 30 zum Zurückhalten der Partikel durchströmt wird. Vorteilhaft lässt sich durch das Auswählen der Porendichte die minimale Größe der Restpartikel bestimmen, weshalb sich diese Filterstufe insbesondere zum Herausfiltern von Feinstaub eignet.

In der Fig. 3 ist eine Schnittdarstellung der bekannten Reinigungsvorrichtung 1 in einer Vertikalebene dargestellt, wobei der im aktiven Betriebszustand der Reinigungsvorrichtung 1 vom erzeugten Saugluftvolumenstrom durchlaufende Saugluftpfad 29 durch Richtungspfeile schematisiert skizziert ist.

Es wurde bereits erwähnt, dass der mit den Partikeln beladene Saugluftvolumenstrom in dem aktiven Betriebszustand der Reinigungsvorrichtung 1 durch die Luftaufnahmeöffnung 2 aus der Umgebung angesaugt wird, wobei die Luftaufnahmeöffnung 2 einen ersten Leitungsquerschnitt 21 umfasst. Über eine hohlzylindrische Zuleitung 33 steht die Luftaufnahmeöffnung 2 mit einem ersten Ende 7 des Tauchrohrs 3 der Flüssigkeitsaufnahmeeinheit 4 in Wirkverbindung.

Das Tauchrohr 3 umfasst einen Umlenkabschnitt 9, der so ausgebildet ist, dass in dem aktiven Betriebszustand der mit den Partikeln beladene Saugluftvolumenstrom in Bezug auf eine horizontale Einströmrichtung R_ein an dem ersten Ende 7 auf eine im Wesentlichen vertikale Ausströmrichtung umgelenkt wird. Ferner umfasst das Tauchrohr 3 einen Absenkabschnitt 10, der sich entlang einer Vertikalachse V erstreckt und der wenigstens bereichsweise in die von der Flüssigkeitsaufnahmeeinheit 4 aufnehmbare Flüssigkeit eintaucht.

Erfindungsgemäß umfasst das Tauchrohr 3 an dem zweiten Ende 8 einen zweiten Leitungsquerschnitt 22, der in Bezug auf den ersten Leitungsquerschnitt 21 der Luftaufnahmeöffnung 2 größer ausgebildet ist. Vorteilhaft führt die Erweiterung des Leitungsquerschnitts von der Luftaufnahmeöffnung 2 zum zweiten Ende 8 des Tauchrohrs 3 zu einem Verringern der Strömungsgeschwindigkeit des Saugluftvolumenstroms. Somit wird die Strömungsgeschwindigkeit des Saugluftvolumenstroms beim Durchströmen der Flüssigkeit reduziert, weshalb die Reinigungswirkung beim Durchströmen der Flüssigkeit verbessert wird. Erfindungsgemäß vorteilhaft werden aufgrund der geringeren Strömungsgeschwindigkeit mehr Partikel aus dem Saugluftvolumenstrom in der Flüssigkeit gebunden.

Ferner wird es aus der Schnittdarstellung der Fig. 3 deutlich, dass der Umlenkabschnitt 9 so ausgebildet ist, dass zum einen eine Erweiterung des Leitungsquerschnitts vom ersten Leitungsquerschnitt 21 auf den zweiten Leitungsquerschnitt 22 erfolgt und zum anderen ein Winkelelement mit einem Umlenkwinkel β von 90° vorgesehen ist, weshalb der erste Leitungsquerschnitt 21, der in einer Vertikalebene ausgerichtet ist, in den zweiten Leitungsquerschnitt 22 übergeht, der in einer Horizontalebene verläuft.

Durch den Umlenkwinkel β von 90° strömt der Saugluftvolumenstrom gegen die innere Mantelfläche des Tauchrohrs 3, weshalb es auch schon zu einer ersten Beeinflussung der Strömungsgeschwindigkeit kommt.

Ferner geht aus der Schnittdarstellung hervor, dass der Absenkabschnitt 10 des Tauchrohrs 3 mantelseitig einen Austrittsbereich 15 aufweist, der zum Ausbilden eines Grobfilters 17 eine Vielzahl an Öffnungen 14 aufweist, wobei die Öffnungen 14 durch eine Vielzahl von stabförmigen Zinken 16 ausgebildet sind. Die Zinken 16 sind zueinander parallel ausgerichtet und erstecken sich längs des Absenkabschnitts 10, wobei zwei unmittelbar benachbarte Zinken 16 die Öffnungen 14 ausbilden, die spaltförmig ausgebildet sind. Da die Zinken 16, die sich längs einer Vertikalachse erstrecken, in einem Zwischenabschnitt zum Steigern der Stabilität des Tauchrohrs 3 verbunden sind, bilden zwei benachbarte Zinken 16 immer zwei spaltartige Öffnungen 14 mit einer Breite von etwa 2mm und einer Länge von etwa 20mm aus, die in Bezug auf eine Vertikalachse zueinander versetzt angeordnet sind.

Vorteilhaft wird durch den Grobfilter 17 eine zweite Filterstufe der Reinigungsvorrichtung 1 ausgebildet, die große Partikel, die nicht durch die 2mm breiten Öffnungen 14 passen, aus dem Saugluftvolumenstrom zurückhält.

Zudem wird durch das entgegen einer Federkraft auf dem Tauchrohr 2 angeordnete Deckelelement 35 eine Wartungsöffnung und/oder Reinigungsöffnung ausgebildet, um vom Grobfilter 17 zurückgehaltene Partikel manuell wieder aus dem Tauchrohr 3 zu entfernen.

Ferner geht aus der Schnittdarstellung auch hervor, dass das Tauchrohr 3 an dem zweiten Ende 8 mit einem Bodenelement 12 der Flüssigkeitsaufnahmeeinheit 4 verbunden ist. Hierfür liegt die Stirnseite 11 des Tauchrohrs 3 an dem zweiten Ende 8 unmittelbar auf dem Bodenelement 12 auf. Somit bildet das Bodenelement 12 eine Barriere für den Saugluftvolumenstrom, die dazu führt, dass der Saugluftvolumenstrom durch die mantelseig ausgebildeten Öffnungen aus dem Tauchrohr 3 strömt.

Nach dem vollständigen Durchströmen der Flüssigkeit trifft der Saugluftvolumenstrom auf den Tropfenabscheider 13, der im Saugluftpfad 28 saugluftleitend hinter der Flüssigkeitsaufnahmeeinheit 4 angeordnet ist, um Flüssigkeitstropfen, die beim Durchströmen der Flüssigkeit vom Saugluftvolumenstrom aufgenommen wurden, wieder vom Saugluftvolumenstrom zu trennen und diese in die Flüssigkeitsaufnahmeeinheit 4 zurückzuführen. Vorteilhaft können hierbei neben den Flüssigkeitstropfen auch weitre Partikel vom Saugluftvolumenstrom getrennt werden, weshalb der Tropfenabscheider 13 eine dritte Reinigungsstufe der Reinigungsvorrichtung 1 realisiert.

Ferner geht aus der Anordnung des Tropfenabscheiders 13 hervor, dass dieser in Bezug auf eine Horizontalebene in einem Fixierwinkel α von 45° angeordnet ist und zusätzlich die unterste Kante des Tropfenabscheiders 13, die sich entlang einer Tiefenachse in die Bildebene erstreckt 13, über der Flüssigkeitsaufnahmeeinheit 4 angeordnet ist, um das Zurückführen der Flüssigkeit in die Flüssigkeitsaufnahmeeinheit 4 zu erleichtern. Somit werden die zurückgewonnen, abgeschiedenen Flüssigkeitstropfen aufgrund der auf sie wirkenden Gewichtskraft in die Flüssigkeitsaufnahmeeinheit 4 zurückgeführt.

Ferner durchströmt der Saugluftvolumenstrom im aktiven Betriebszustand der Reinigungsvorrichtung 1 schließlich auch noch die Filtereinheit 27, die saugluftleitend zwischen den Unterdruckerzeugungsmitteln 6 und dem Tropfenabscheider 13 angeordnet ist. Die Filtereinheit 27 ermöglicht das Herausfiltern von vom Saugluftvolumenstrom mitgeführten Feinpartikeln, was sich vorteilhaft durch eine Wahl einer Porendichte des Gewebefilters 30 gezielt beeinflussen lässt.

Zum Ausrichten und Fixieren des Gewebefilters 30 umfasst die Filtereinheit 27 die Aufnahmeeinheit 28, die insbesondere zur klemmenden Aufnahme des Gewebefilters 30 ausgebildet ist. Vorteilhaft lässt sich somit eine vierte Reinigungsstufe der Reinigungsvorrichtung 1 realisieren.

Nach dem Durchströmen des Gewebefilters 30 durchströmt der Saugluftvolumenstrom eine Passage, die bevorzugt durch UVC-Lichtstrahlen, die mittels UVC-Lichterzeugungsmitteln 34 erzeugt werden, ausgeleuchtet werden. Vorteilhaft führen die UVC-Lichtstrahlen zu einem Abtöten von vom Saugluftvolumenstrom mitgeführten Viren und Keimen, weshalb hierdurch eine fünfte Reinigungsstufe der Reinigungsvorrichtung 1 realisiert werden kann.

Nach dem Durchströmen der beleuchteten Passage erreicht der Saugluftvolumenstrom die Unterdruckerzeugungsmittel 6, die vorliegend durch einen rotierenden Elektromotor ausgebildet sind. Durch die bereits bekannten Luftabgabeöffnungen 31 in den Seitenflächen 23, 24 wird der gesäuberte Saugluftvolumenstrom dann wieder an die Umgebung abgegeben.

Ferner wird noch darauf hingewiesen, dass die Unterdruckerzeugungsmittel 6 in einem oberen Bereich der Reinigungsvorrichtung 1 angeordnet sind. Vorteilhaft lässt sich somit eine räumliche Trennung zwischen dem mit der Flüssigkeit in Kontakt kommenden unteren Bereich und dem aufgrund der verbauten Elektronik feuchtigkeitsempfindlichen oberen Bereich der Reinigungsvorrichtung 1 realisieren.

In der Fig. 4 ist eine zweite Schnittansicht der bekannten Reinigungsvorrichtung 1 entlang einer Horizontalebene dargestellt.

Aus der Darstellung geht der ausgangsseitige Verlauf des Saugluftvolumenstroms entlang des skizzierten Saugluftpfads 29 hervor, der zunächst über die Saugluftvolumenstromabführöffnung 37 und dann über die im Gehäuse durch die Perforierung ausgebildete Luftabgabeöffnung 31 in den gegenüberliegenden Seitenflächen 23, 24 an die Umgebung abgegeben werden.

Ferner ist schematisiert auch ein Kühlluftkreislauf der Unterdruckerzeugungsmittel 6 dargestellt, wobei Umgebungsluft zum Kühlen der Unterdruckerzeugungsmittel 6 durch die Lüftungsöffnung 36 angesaugt wird und durch die umfangsseitig der Unterdruckerzeugungsmittel 6 angeordneten Kühlkreislaufableitöffnungen 38 und die Luftabgabeöffnung 31 wieder an die Umgebung abgeleitet wird.

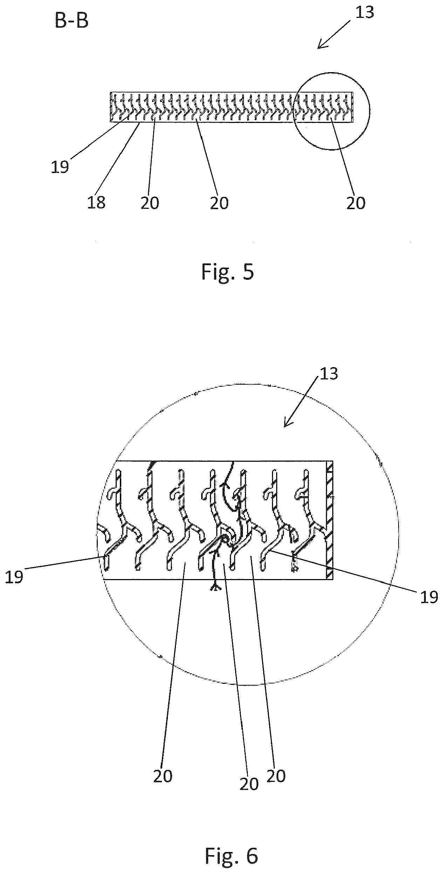

In der Fig. 5 ist eine Schnittansicht des Tropfenabscheiders 13 nach einer in der Fig. 3 angedeuteten Perspektive B dargestellt.

Aus der Schnittansicht wird zunächst der sich in einer Ebene erstreckende Anströmbereich 18 deutlich, von dem eine Vielzahl von profilierten, voneinander beabstandeten Lamellen 19 ausgebildet sind, wobei zwischen zwei unmittelbar benachbarten Lamellen 19 jeweils ein Durchströmkanal 20 zum Aufnehmen eines Teilstroms des auf den Anströmbereich 18 frontal treffenden Saugluftvolumenstroms ausgebildet wird.

Die blechartigen Lamellen 19 weisen entlang der Durchströmkanäle 20 in der vorgegebenen Durchströmrichtung bogenförmige Profilierungen auf, die den Teilströmen Richtungsänderungen aufzwingen und Unterdruckbereich erzeugen. Durch die Richtungsänderungen erfahren die mitgeführten Flüssigkeitstropfen aufgrund der massebedingten Trägheit eine Zentrifugalkraft, die zum Absorbieren der Flüssigkeit sowie auch von mitgeführten Partikeln (Restpartikeln) führt.

In der Fig. 6 ist der in der Fig. 5 umkreiste Bereich im vergrößerten Maßstab dargestellt. Ferner ist der Verlauf eines Teilstroms des Saugluftvolumenstroms beim Durchströmen der Durchströmkanäle 20 des Tropfenabscheiders 13 schematisiert skizziert.

Aus der schematisierten Darstellung geht hervor, dass durch die bogenförmige Profilierung Umlenkbereiche für die Teilströme ausgebildet werden, weshalb die Teilströme beim Durchströmen der Durchströmkanäle 20 wenigstens zwei Richtungsänderungen aufgezwungen werden, die zum Ausscheiden der mitgeführten Flüssigkeitstropfen und Restpartikeln führen.

Schließlich wird mit Bezug auf die Fig. 3 noch darauf hingewiesen, dass der Anströmbereich 18 des Flüssigkeitsabsorbers 13 in Bezug auf den zweiten Leitungsquerschnitt 22 des Tauchrohrs 3 klein und/oder kleiner gewählt ist. Vorteilhaft kann somit die Strömungsgeschwindigkeit des Saugluftvolumenstroms beim Auftreffen auf den Flüssigkeitsabsorber 13 wieder erhöht werden, um somit die auf die Flüssigkeitstropfen und/oder Restpartikel wirkende Zentrifugalkraft auch zu erhöhen. Somit kann vorteilhaft ein großer Anteil der mitgeführten Flüssigkeitstropfen vom Saugluftvolumenstrom getrennt werden und wieder in die Flüssigkeitsaufnahmeeinheit zurückgeführt werden.

### Bezugszeichenliste

- 1: Reinigungsvorrichtung
- 2: Luftaufnahmeöffnung
- 3: Tauchrohr
- 4: Flüssigkeitsaufnahmeeinheit
- 5: oberes Gehäuseelement
- 6: Unterdruckerzeugungsmittel
- 7: erstes Ende des Tauchrohrs
- 8: zweiten Ende des Tauchrohrs
- 9: Umlenkabschnitt
- 10: Absenkabschnitt
- 11: Stirnseite
- 12: Bodenelement
- 13: Tropfenabscheider
- 14: Öffnung
- 15: Austrittsbereich
- 16: Zinken
- 17: Grobfilter
- 18: Anströmbereich
- 19: Lamelle
- 20: Durchströmkanal
- 21: erster Leitungsquerschnitt
- 22: zweiter Leitungsquerschnitt
- 23: Seitenfläche (erste)
- 24: Seitenfläche (zweite)
- 25: Verschlussmittel
- 26: unteres Gehäuseelement
- 27: Filtereinheit
- 28: Aufnahmeeinheit für einen Gewebefilter
- 29: Saugluftpfad
- 30: Gewebefilter
- 31: Luftabgabeöffnung
- 32: Vorderseite
- 33: Zuleitung
- 34: UVC-Lichterzeugungsmittel
- 35: Deckelelement des Tauchrohrs
- 36: Lüftungsöffnung Kühlkreislauf Zuluft
- 37: Saugluftvolumenstromabführöffnung Unterdruckerzeugungsmittel
- 38: Kühlkreislaufableitöffnung Unterdruckerzeugungsmittel

- R_ein: Einströmrichtung
- V: Vertikalachse
- H: Horizontalachse

- α: Fixierwinkel
- β: Umlenkwinkel

## Patentansprüche

1. Reinigungsvorrichtung (1) zum Ansaugen eines mit Partikeln beladenen Saugluftvolumenstroms aus einer Umgebung, aufweisend eine einen ersten Leitungsquerschnitt (21) umfassende Luftaufnahmeöffnung (2) zum Ansaugen des mit den Partikeln, Aerosolen, Bakterien und/oder Viren beladenen Saugluftvolumenstroms aus der Umgebung in einem aktiven Betriebszustand der Reinigungsvorrichtung (1),
eine ein Tauchrohr (3) umfassende Flüssigkeitsaufnahmeeinheit (4) zum Aufnehmen von einer Flüssigkeit, insbesondere von Wasser,
wobei das Tauchrohr (3) an einem ersten Ende (7) mit der Luftaufnahmeöffnung (2) saugluftleitend verbunden ist und so ausgebildet ist, dass es an einem zweiten Ende (8) in die aufnehmbare Flüssigkeit, insbesondere in das Wasser, eintaucht, derartig, dass in dem aktiven Betriebszustand der Reinigungsvorrichtung (1) die aufnehmbare Flüssigkeit von dem mit den Partikeln beladenen Saugluftvolumenstrom zum Zurückhalten der Partikel durchströmt wird, sowie
Unterdruckerzeugungsmittel (6) zum Erzeugen des Saugluftvolumenstroms in dem aktiven Betriebszustand wobei ein zweiter Leitungsquerschnitt (22) des Tauchrohrs (3) zum Führen des Saugluftvolumenstroms wenigstens an dem zweiten Ende (8) größer ist als der erste Leitungsquerschnitt (21) der Luftaufnahmeöffnung (2) **dadurch gekennzeichnet,**
**dass** das Tauchrohr (3), insbesondere im Bereich des Absenkabschnitts (10), zum Ausbilden eines Grobfilters (17) einen Austrittsbereich (15) mit einer Vielzahl an Öffnungen (14) aufweist, wobei die Öffnungen (14) bevorzugt durch eine Vielzahl von stabförmigen Zinken (16), die insbesondere zueinander parallel angeordnet und/oder ausgerichtet sind, weiter bevorzugt schlitz- und/oder spaltartig, ausgebildet sind.

2. Vorrichtung nach Anspruch 1,
**dadurch gekennzeichnet,**
**dass** ein Verhältnis zwischen dem zweiten Leitungsquerschnitt (22) des Tauchrohrs (3) und dem ersten Leitungsquerschnitt (21) der Luftaufnahmeöffnung (2) wenigstens 2, bevorzugt wenigstens 3, weiter bevorzugt wenigstens 3,5 beträgt.

3. Vorrichtung nach Anspruch 1 oder 2,
**dadurch gekennzeichnet,**
**dass** das Tauchrohr (3) wenigstens einen bogen- und/oder winkelförmigen Umlenkabschnitt (9) und einen, insbesondere vertikal ausgerichteten, Absenkabschnitt (10) umfasst,
wobei der Umlenkabschnitt (9) so ausgebildet ist, dass in dem aktiven Betriebszustand der mit den Partikeln beladene Saugluftvolumenstrom in Bezug auf eine Einströmrichtung (R_ein) umgelenkt und verwirbelt wird
und wobei der Absenkabschnitt (10) so ausgebildet ist, dass der in dem aktiven Betriebszustand im Umlenkabschnitt (9) umgelenkte und verwirbelte Saugluftvolumenstrom durch eine Erweiterung des Leitungsquerschnitts in Bezug auf eine mittlere Einströmgeschwindigkeit verlangsamt wird.

4. Vorrichtung nach Anspruch 3,
**dadurch gekennzeichnet,**
**dass** der Umlenkabschnitt (9) so ausgebildet ist, dass in dem aktiven Betriebszustand der mit den Partikeln beladene Saugluftvolumenstrom in Bezug auf die Einströmrichtung (R_ein), insbesondere in Bezug auf eine Horizontalachse, um einen Umlenkwinkel (β) von größer als 60°, bevorzugt größer als 70°, besonders bevorzugt größer als 80°, ganz besonders bevorzugt etwa 90° umgelenkt wird.

5. Vorrichtung nach Anspruch 3 oder 4,
**dadurch gekennzeichnet,**
**dass** das Tauchrohr (3) so in der Flüssigkeitsaufnahmeeinheit (4) angeordnet ist, dass der Absenkabschnitt (10) sich entlang einer Vertikalachse (V) erstreckt, derartig, dass in dem aktiven Betriebszustand der Saugluftvolumenstrom vertikal absinkt und somit insbesondere im Wesentlichen quer, insbesondere im rechten Winkel, zu der im Ruhezustand der Reinigungsvorrichtung (1) von der aufnehmbaren Flüssigkeit ausbildbaren Flüssigkeitsoberfläche in die Flüssigkeit einströmt.

6. Vorrichtung nach Anspruch 1,
**dadurch gekennzeichnet,**
**dass** das Tauchrohr (3) an dem zweiten Ende (8) eine Stirnseite (11) aufweist, die mit einem Bodenelement (12) der Flüssigkeitsaufnahmeeinheit (4) verbunden ist, derartig, dass in dem aktiven Betriebszustand der Saugluftvolumenstrom ausschließlich durch die Öffnungen (14) aus dem Tauchrohr (3) strömt.

7. Vorrichtung nach einem der vorangehenden Ansprüche,
**dadurch gekennzeichnet,**
**dass** die Reinigungsvorrichtung (1) einen Tropfenabscheider (13) umfasst, der zum Zurückhalten von vom Saugluftvolumenstrom aufgenommenen Flüssigkeitstropfen und weiteren Partikeln saugluftleitend zwischen der Flüssigkeitsaufnahmeeinheit (4) und den Unterdruckerzeugungsmitteln (6) angeordnet ist.

8. Vorrichtung nach Anspruch 7,
**dadurch gekennzeichnet,**
**dass** der Tropfenabscheider (13) einen Anströmbereich (18) umfasst, der durch eine Vielzahl von profilierten, voneinander beabstandeten, insbesondere blechartigen, Lamellen (19) ausgebildet ist, wobei zwischen zwei benachbarten Lamellen (19) jeweils ein Durchströmkanal (20) mit wenigstens zwei Umlenkbereichen für den Saugluftvolumenstrom ausgebildet ist und/oder wobei der Anströmbereich (18) in einem Fixierwinkel (α) zwischen 20° bis 70°, bevorzugt 30° bis 60°, besonders bevorzugt 40° bis 50°, ganz besonders bevorzugt im Wesentlichen 45° zu einer Horizontalebene (H1) ausgerichtet ist.

9. Vorrichtung nach Anspruch 7 oder 8,
**dadurch gekennzeichnet,**
**dass** der Tropfenabscheider (13) statisch und/oder unbeweglich, insbesondere aus nicht-rotierenden Elementen ausgebildet ist.

10. Vorrichtung nach einem der vorhergehenden Ansprüche,
**dadurch gekennzeichnet,**
**dass** die Reinigungsvorrichtung (1) eine Filtereinheit (27) zum Herausfiltern von vom Saugluftvolumenstrom mitgeführten Feinpartikeln umfasst, die insbesondere in einem in der Reinigungsvorrichtung (1) vom Saugluftvolumenstrom im aktiven Betriebszustand ausgebildeten Saugluftpfad (29) saugluftleitend zwischen dem Tropfenabscheider (13) und den Unterdruckerzeugungsmitteln (6) angeordnet ist.

11. Vorrichtung nach Anspruch 10,
**dadurch gekennzeichnet,**
**dass** die Filtereinheit (27) eine Aufnahmeeinheit (28) für einen austauschbaren Gewebefilter (30) umfasst, wobei die Aufnahmeeinheit (28) so ausgebildet ist, dass der Gewebefilter (30) im aktiven Betriebszustand vom Saugluftvolumenstrom zum Zurückhalten der Feinpartikeln durchströmt wird.

12. Vorrichtung nach einem der vorhergehenden Ansprüche,
**dadurch gekennzeichnet,**
**dass** die Reinigungsvorrichtung (1) UV- und/oder UVC-Lichterzeugungsmittel (34) umfasst, die so in der Reinigungsvorrichtung (1) angeordnet sind, dass der Saugluftpfad (28) bereichsweise, insbesondere entlang einer Passage des Saugluftpfads (29), ausgeleuchtet wird, derartig, dass der im aktiven Betriebszustand erzeugbare Saugluftvolumenstrom zum Abtöten von mitgeführten Viren und/oder Keimen vollständig beleuchtet wird.

13. Verwendung der erfindungsgemäßen Reinigungsvorrichtung (1) als Umgebungsluftreinigungsvorrichtung.

14. Verwendung der erfindungsgemäßen Reinigungsvorrichtung (1) als Nassstaubsauger.

## Claims

1. A cleaning apparatus (1) for suctioning suctioned-air volume flow laden with particles from an environment, the cleaning apparatus (1) having an air reception opening (2) comprising a first tube cross section (21) and serving to suction the suctioned-air volume flow laden with the particles, aerosols, bacteria and/or viruses from the environment in an active operating state of the cleaning apparatus (1),
a liquid reception unit (4) comprising a dip pipe (3) and serving to receive a liquid, in particular water, the dip pipe (3) being connected to the air reception opening (2) at a first end (7) in an air-suctioning manner and being designed such that it dips into the liquid to be received, in particular into the water, at a second end (8) in such a manner that the suctioned-air volume flow laden with the particles flows through the receivable liquid in the active operating state of the cleaning apparatus (1) in order to retain the particles, and
a negative pressure generator (6) for generating the suctioned-air volume flow in the active operating state, a second tube cross section (22) of the dip tube (3) for guiding the suctioned-air volume flow being larger at least at the second end (8) than the first tube cross section (21) of the air reception opening (2),
**characterized in that**
the dip tube (3), in particular in the area of the lowering section (10), has an exit area (15) having a plurality of openings (14) for forming a coarse filter (17), the openings (14) being designed preferably by a plurality of rod-shaped tines (16), which are in particular disposed and/or aligned parallel to each other, further preferably designed in a slit or gap-like manner.

2. The apparatus according to claim 1,
**characterized in that**
a ratio between the second tube cross section (22) of the dip tube (3) and the first tube cross section (21) of the air reception opening (2) is at least 2, preferably at least 3, further preferably at least 3.5.

3. The apparatus according to claim 1 or 2,
**characterized in that**
the dip tube (3) comprises at least one arced and/or angled deflection section (9) and a, in particular vertically aligned, lowering section (10),
the deflection section (9) being designed such that the suctioned-air volume flow is deflected and whirled with respect to an influx direction (R_ein) in the active operating state
and the lowering section (10) being designed such that the suctioned-air volume flow deflected and whirled in the deflection section (9) in the active operating state is decelerated with respect to a medium influx speed by the tube cross section becoming larger.

4. The apparatus according to claim 3,
**characterized in that**
the deflection section (9) is designed such that the suctioned-air volume flow laden with the particles is deflected in the active operating state by a deflection angle (β) greater than 60°, preferably greater than 70°, particularly preferably greater than 80°, even more preferably approximately 90°, in relation to an influx direction (R_ein), in particular in relation to a horizontal axis.

5. The apparatus according to claim 3 or 4,
**characterized in that**
the dip tube (3) is disposed such in the liquid reception unit (4) that the sink section (10) extends in such a manner along a vertical axis (V) that the suctioned-air volume flow sinks vertically in the active operating state and thus flows into the liquid in particular essentially transversely, in particular on the square, to the liquid surface formable by the receivable liquid in the idle state of the cleaning apparatus (1).

6. The apparatus according to claim 1,
**characterized in that**
the dip tube (3) has a front face (11) on the second end (8), the front face (11) being connected in such a manner to a bottom element (12) of the liquid reception unit (4) that the suctioned-air volume flow flows from the dip tube (3) only through the openings (14) in the active operating state.

7. The apparatus according to any one of the preceding claims,
**characterized in that**
the cleaning apparatus (1) comprises a droplet separator (13) which is disposed in an air-suctioning manner between the liquid reception unit (4) and the negative pressure generator (6) for retaining liquid droplets and other particles received by the suctioned-air volume flow.

8. The apparatus according to claim 7,
**characterized in that**
the droplet separator (13) comprises a flow area (18), which is formed by a plurality of profiled, spaced-apart, in particular sheet-like lamellae (19), a flow channel (20) having at least two deflection areas for the suctioned-air volume flow being formed between two adjacent lamellae (19) and/or
the flow area (18) being aligned in a fixating angle (α) between 20° to 70°, preferably 30° to 60°, particularly preferably 40° to 50°, even more preferably essentially at 45°, to a horizontal plane (H1).

9. The apparatus according to claim 7 or 8,
**characterized in that**
the droplet separator (13) is static and/or immovable, in particular made of non-rotatable elements.

10. The apparatus according to any one of the preceding claims,
**characterized in that**
the cleaning apparatus (1) comprises a filter unit (27) for filtering fine particles carried along by the suctioned-air volume flow, the filter unit (27) being disposed in an air-suctioning manner between the droplet separator (13) and the negative pressure generator (6) in particular in an suctioned-air path (29), which is formed by the suctioned-air volume flow in the cleaning apparatus (1) in the active operating state.

11. The apparatus according to claim 10,
**characterized in that**
the filter unit (27) comprises a reception unit (28) for an exchangeable tissue filter (30), the reception unit (28) being designed such that the suctioned-air volume flow flows through the tissue filter (30) in the active operating state in order to retain the fine particles.

12. The apparatus according to any one of the preceding claims,
**characterized in that**
the cleaning apparatus (1) comprises UV and/or UVC light generators (34), which are disposed such in the cleaning apparatus (1) that sections of the suctioned-air path (28), in particular along a pass of the suctioned-air path (29), are illuminated in such a manner that the suctioned-air volume flow generatable in the active operating state is entirely illuminated for killing carried-along viruses and/or germs.

13. A use of the cleaning apparatus (1) according to the invention as an ambient-air cleaning apparatus.

14. A use of the cleaning apparatus (1) according to the invention as a wet vacuum cleaner.

## Revendications

1. Appareil de nettoyage (1) pour aspirer un débit volumétrique de l'air aspiré chargé des particules à partir d'un environnement, l'appareil de nettoyage (1) ayant une ouverture (2) de réception de l'air comprenant une première section transversale de tube (21) et servant à aspirer le débit volumétrique de l'air aspiré chargé de particules, des aérosols, des bactéries, et/ou des virus de l'environnement dans un état d'opération actif de l'appareil de nettoyage (1),
une unité (4) de réception de liquide comprenant un tube plongeur (3) et servant à recevoir un liquide, notamment l'eau, le tube plongeur (3) étant relié à l'ouverture (2) de réception de l'air à une première extrémité (7) dans une manière à aspirer l'air et étant configuré de telle manière qu'il plonge dans le liquide à être reçu, notamment dans l'eau, à une deuxième extrémité (8) de telle manière que le débit volumétrique de l'air aspiré chargé de particules s'écoule par le liquide à être reçu dans l'état d'opération actif de l'appareil de nettoyage (1) afin de retenir les particules, et
un générateur de vide (6) pour la génération du débit volumétrique de l'air aspiré dans l'état d'opération actif, une deuxième section transversale (22) du tube plongeur (3) pour le guidage du débit volumétrique de l'air aspiré étant plus grande à la deuxième extrémité (8) que la première section transversale de tube (21) de l'ouverture (2) de réception de l'air,
**caractérisé en ce que**
le tube plongeur (3), notamment dans la zone de la partie abaisseuse (10), a une zone de sortie (15) ayant une pluralité d'ouvertures (14) pour la formation d'un filtre grossier (17), les ouvertures (14) étant formées de préférence par une pluralité des fourchons (16) en forme de barre, qui sont notamment disposés et/ou orientés parallèlement l'un à l'autre, de préférence particulière configurés de manière d'une fente et/ou d'une fissure.

2. Appareil selon la revendication 1,
**caractérisé en ce**
**qu'**un rapport entre la deuxième section transversale (22) du tube plongeur (3) et de la première section transversale de tube (21) de l'ouverture (2) de réception de l'air est au moins 2, de préférence au moins 3, de préférence particulière au moins 3.5.

3. Appareil selon la revendication 1 ou la revendication 2,
**caractérisé en ce que**
le tube plongeur (3) comprend au moins une partie de déviation (9) arquée et/ou angulaire et une partie abaisseuse (10), notamment orientée verticalement,
la partie de déviation (9) étant configurée de telle manière que le débit volumétrique de l'air aspiré est dévié et tourbillonné par rapport à un sens d'afflux (R_ein) dans l'état d'opération actif
et la partie abaisseuse (10) étant configurée de telle manière que le débit volumétrique de l'air aspiré dévié et tourbillonné dans la partie de déviation (9) dans l'état d'opération actif est décéléré par rapport à une vitesse d'afflux moyenne en élargissant la section transversale de tube.

4. Appareil selon la revendication 3,
**caractérisé en ce que**
la partie de déviation (9) est configurée de telle manière que le débit volumétrique de l'air aspiré chargé de particules est dévié dans l'état d'opération actif par un angle de déviation (β) plus grand de 60°, de préférence plus grand de 70°, de préférence particulière plus grand de 80°, de préférence encore davantage d'environ 90°, en relation au sens d'afflux (R_ein), notamment en relation d'un axe horizontal.

5. Appareil selon la revendication 3 ou la revendication 4,
**caractérisé en ce que**
le tube plongeur (3) est disposé dans l'unité (4) de réception de liquide de telle manière que la partie abaisseuse (10) s'étend le long un axe vertical (V) de telle manière que le débit volumétrique de l'air aspiré s'abaisse verticalement dans l'état d'opération actif et donc s'écoule dans le liquide notamment essentiellement transversalement, notamment à angle droit, à la surface de liquide formable par le liquide à être reçu à l'état de repos de l'appareil de nettoyage (1).

6. Appareil selon la revendication 1,
**caractérisé en ce que**
le tube plongeur (3) a une face frontale (11) sur la deuxième extrémité (8), la face frontale (11) étant reliée à un élément de fond (12) de l'unité (4) de réception de liquide de telle manière que le débit volumétrique de l'air aspiré s'écoule à partir du tube plongeur (3) seulement par les ouvertures (14) dans l'état d'opération actif.

7. Appareil selon l'une quelconque des revendications précédentes,
**caractérisé en ce que**
l'appareil de nettoyage (1) comprend un séparateur de gouttelettes (13) qui est disposé dans une manière à aspirer l'air entre l'unité (4) de réception de liquide et le générateur de vide (6) pour la rétention de gouttelettes de liquide et des outres particules reçues par le débit volumétrique de l'air aspiré.

8. Appareil selon la revendication 7,
**caractérisé en ce que**
le séparateur de gouttelettes (13) comprend une partie d'écoulement (18), qui est formée par une pluralité de lamelles (19) profilées espacées, notamment semblable à la tôle, un canal d'écoulement (20) ayant au moins deux parties de déviation pour le débit volumétrique de l'air aspiré étant formé entre deux lamelles (19) adjacentes à chaque fois et/ou
la partie d'écoulement (18) étant orientée à un angle de fixation (a) entre 20° à 70°, de préférence entre 30° à 60°, de préférence particulière entre 40° à 50°, de préférence encore davantage essentiellement à 45° à un plan horizontal (H1).

9. Appareil selon la revendication 7 ou la revendication 8,
**caractérisé en ce que**
le séparateur de gouttelettes (13) est statique et/ou immobile, notamment fait d'éléments non rotatifs.

10. Appareil selon l'une quelconque des revendications précédentes,
**caractérisé en ce que**
l'appareil de nettoyage (1) comprend une unité de filtre (27) pour la filtration des particules fines amenées par le débit volumétrique de l'air aspiré, l'unité de filtre (27) étant disposée de manière à aspirer l'air entre le séparateur de gouttelettes (13) et le générateur de vide (6) notamment dans un trajet d'air aspiré (29), qui est formé par le débit volumétrique de l'air aspiré dans l'appareil de nettoyage (1) dans l'état d'opération actif,.

11. Appareil selon la revendication 10,
**caractérisé en ce que**
l'unité de filtre (27) comprend une unité de réception (28) pour un filtre en tissu (30) échangeable, l'unité de réception (28) étant configurée de telle manière que le débit volumétrique de l'air aspiré s'écoule par le filtre en tissu (30) dans l'état d'opération actif afin de retenir les particules fines.

12. Appareil selon l'une quelconque des revendications précédentes,
**caractérisé en ce que**
l'appareil de nettoyage (1) comprend des générateurs de lumière UV et/ou UVC (34), qui sont disposés dans l'appareil de nettoyage (1) de telle manière que des parties du trajet d'air aspiré (28), notamment le long un passage du trajet d'air aspiré (29), sont illuminées de telle manière que le débit volumétrique de l'air aspiré générable dans l'état d'opération actif est illuminé entièrement pour la destruction de virus et/ou germes amenés.

13. Utilisation de l'appareil de nettoyage (1) selon l'invention comme appareil de nettoyage à l'air ambient.

14. Utilisation de l'appareil de nettoyage (1) selon l'invention comme aspirateur de liquides.
